(19) **Europäisches Patentamt / European Patent Office / Office européen des brevets**

(11) **EP 2 667 235 A1**

(12) **EUROPEAN PATENT APPLICATION**
published in accordance with Art. 153(4) EPC

(43) Date of publication:
**27.11.2013 Bulletin 2013/48**

(51) Int Cl.:
**G02B 23/26** (2006.01)     **A61B 1/00** (2006.01)

(21) Application number: **12844509.5**

(22) Date of filing: **19.10.2012**

(86) International application number:
**PCT/JP2012/077082**

(87) International publication number:
**WO 2013/061874 (02.05.2013 Gazette 2013/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2011 JP 2011237111**

(71) Applicant: **OLYMPUS MEDICAL SYSTEMS CORP. Tokyo 151-0072 (JP)**

(72) Inventors:
 • **MORITA, Kazuo**
  **Shibuya-ku, Tokyo 151-0072 (JP)**
 • **HOMMA, Hiroyuki**
  **Shibuya-ku, Tokyo 151-0072 (JP)**

(74) Representative: **Schicker, Silvia**
 **Wuesthoff & Wuesthoff**
 **Patent- und Rechtsanwälte**
 **Schweigerstrasse 2**
 **81541 München (DE)**

(54) **ENDOSCOPE ILLUMINATION OPTICAL SYSTEM**

(57)     Illumination light emitted from a light source is efficiently distributed to an observation target, and luminous intensity distribution performance is enhanced. An endoscopic illumination optical system, including a light guide bundle 10 for guiding illumination light emitted from a light source, the light guide bundle being provided in an insertion portion of an endoscope device, and an illumination lens 11 for distributing the illumination light emitted from the light guide bundle to an observation target, wherein the illumination lens includes two points of inflection P in a vertical cross section including an optical axis of the lens, and includes a first concave surface 11a that is located on an inner side of the two points of inflection, and an annular second concave surface 11b that is located on an outer side of the two points of inflection and that is continuous with an outer periphery of the first concave surface, the second concave surface being arranged so as to face an outer peripheral edge of the light guide bundle in an optical axis direction.

FIG. 1

EP 2 667 235 A1

**Description**

{Technical Field}

The present invention relates to an illumination optical system to be applied to an endoscope device.

{Background Art}

Generally, an illumination optical system to be applied to an endoscope device includes a light guide bundle, which is a plurality of light guide fibers bound together, and a concave lens for illumination arranged in front of an illumination light emission end of the light guide bundle, and in the case of observing the inside of a body cavity by an endoscope device, illumination light emitted from a light source is guided by the light guide bundle, and the guided illumination light is diffused by the concave lens and distributed to an observation target region.
As an example of such an illumination optical system, Patent Literature 1 discloses one that uses a light guide bundle and one concave lens, for example.

{Citation List}

{Patent Literature}

{PTL 1}
Japanese Unexamined Patent Application, Publication No. Hei10-288742

{Summary of Invention}

{Technical Problem}

However, with such an illumination optical system, illumination light emitted from a light guide fiber at a peripheral portion of a light guide bundle arranged near a concave lens is strongly refracted in a direction away from an optical axis at the time of passing through a concave surface at an outer peripheral portion of the concave lens. That is, the refracted illumination light hits a side surface without being emitted from a front end surface of the concave lens, and, as a result, the proportion of illumination light reaching an observation target to the entire illumination light is reduced, and sufficient luminous intensity distribution performance is not obtained.
The present invention is made in view of the circumstance described above, and its object is to provide an endoscopic illumination optical system capable of efficiently distributing illumination light emitted from a light source to an observation target and thereby enhancing the luminous intensity distribution performance.

{Solution to Problem}

To achieve the object described above, the present invention provides the following solution.
An aspect of the present invention provides an endoscopic illumination optical system, including a light guide bundle for guiding illumination light emitted from a light source, the light guide bundle being provided in an insertion portion of an endoscope device, and an illumination lens for distributing the illumination light emitted from the light guide bundle to an observation target, wherein the illumination lens includes two points of inflection in a vertical cross section including an optical axis of the lens, and includes a first concave surface that is located on an inner side of the two points of inflection, and an annular second concave surface that is located on an outer side of the two points of inflection and that is continuous with an outer periphery of the first concave surface, the second concave surface being arranged so as to face an outer peripheral edge of the light guide bundle in an optical axis direction.
According to the present invention, illumination light emitted from the light guide fiber located near the center of the light guide bundle is diffused by the first concave surface located in the center of the illumination lens. On the other hand, illumination light emitted from the light guide fiber located at a peripheral portion of the light guide bundle is refracted by the second concave surface facing the optical axis direction. The direction of the curve of the second concave surface is opposite to that of the first concave surface with the points of inflection as the boundary, and thus, illumination light incident on the second concave surface is refracted in a direction toward the center of the illumination lens.
In the present invention, wherein a distance A between the two points of inflection in the vertical cross section including the optical axis and a diameter dimension D of an end surface of the light guide bundle satisfy a following condition (1) is preferable:

$$0.75 \leq A/D \leq 0.85 \quad ...(1).$$

By satisfying the condition (1), illumination light may be further diffused, and desirable luminous intensity

In the present invention,wherein a curvature radius R1 of the first concave surface and a curvature radius R2 of the second concave surface satisfy a following condition (2) is preferable:

$$0.25 \leq |R2/R1| \leq 0.35 \quad ...(2).$$

By satisfying the condition (2), the refraction of illumination light emitted from a light guide fiber at a peripheral portion of the light guide bundle in a direction away from the optical axis may be reduced while maintaining the diameter of the illumination lens to be small, and the luminous intensity distribution performance may be further enhanced.

{Effect of the invention}

According to the present invention,an endoscopic illumination optical system is capable of efficiently distributing illumination light emitted from a light source to an observation target and thereby enhancing the luminous intensity distribution performance.

{Brief Description of Drawings}

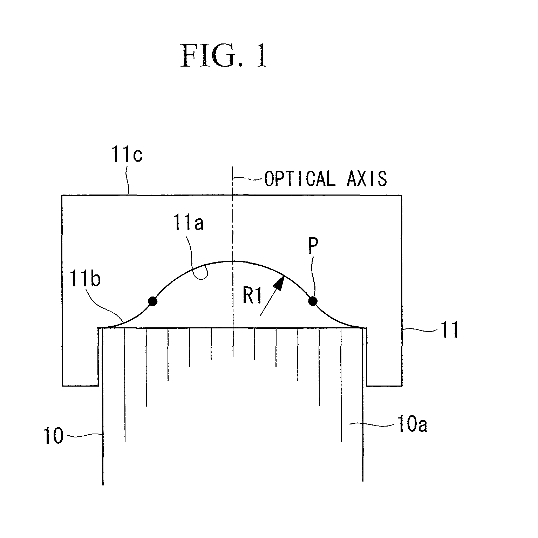

{Fig. 1}
Fig. 1 is a schematic configuration diagram showing a vertical cross section of an endoscopic illumination optical system according to an embodiment of the present invention.
{Fig. 2}
Fig. 2 is a diagram showing luminous intensity distribution characteristic (a relationship between an illuminance ratio and an angle) of an endoscopic illumination optical system according to an embodiment of the present invention.
{Fig. 3}
Fig. 3 is a diagram showing a comparison table of illumination efficiency of an endoscopic illumination optical system according to an embodiment of the present invention.
{Fig. 4}
Fig. 4 is a schematic configuration diagram showing a vertical cross section of an endoscopic illumination optical system according to an embodiment of the present invention where |R2/R1| is 0.05, which is lower than a condition (2).
{Fig. 5}
Fig. 5 is a schematic configuration diagram showing a vertical cross section of an endoscopic illumination optical system according to an embodiment of the present invention where |R2/R1| is 0.5, which is greater than the condition (2).
{Fig. 6}
Fig. 6 is a schematic configuration diagram showing a vertical cross section of an endoscopic illumination optical system according to Example 1 of the present invention.
{Fig. 7}
Fig. 7 is a schematic configuration diagram showing a vertical cross section of an endoscopic illumination optical system according to Example 2 of the present invention.
{Fig. 8}
Fig. 8 is a schematic configuration diagram showing a vertical cross section of an endoscopic illumination optical system according to Example 3 of the present invention.
{Fig. 9}
Fig. 9 is a schematic configuration diagram showing a vertical cross section of an endoscopic illumination optical system according to Example 4 of the present invention.
{Fig. 10}
Fig. 10 is a schematic configuration diagram showing a vertical cross section of an endoscopic illumination optical system according to Example 5 of the present invention.
{Fig. 11}

Fig. 11 is a diagram showing values of A/D and values of |R2/R1| of the illumination optical systems for endoscopes of Examples 1 to 5 of the present invention.

{Description of Embodiments}

Hereinafter, an endoscopic illumination optical system according to an embodiment of the present invention will be described with reference to the drawings.

Fig. 1 shows a vertical cross section including an optical axis of the illumination optical system 1 for endoscopes according to the present embodiment. As shown in Fig. 1, an illumination optical system 1 for endoscopes includes a light guide bundle 10, which is a plurality of light guide fibers 10a for guiding illumination light emitted from a light source not shown bound together, and an  illumination lens 11 for distributing illumination light emitted from an end surface of the light guide bundle 10 to an observation target.

The illumination lens 11 includes, in a vertical cross section including the optical axis of the lens 11, two points of inflection P, a first concave surface 11a that is located on the inner side of the two points of inflection P, and an annular second concave surface 11b that is located on the outer side of the points of inflection P and that is continuous with an outer periphery of the first concave surface 11a.

With the illumination lens 11, a distance A between the two points of inflection P and a diameter dimension D of an end surface of the light guide bundle 10 satisfy the following condition (3).

$$0.75 \leq A/D \leq 0.85 \quad \ldots (3)$$

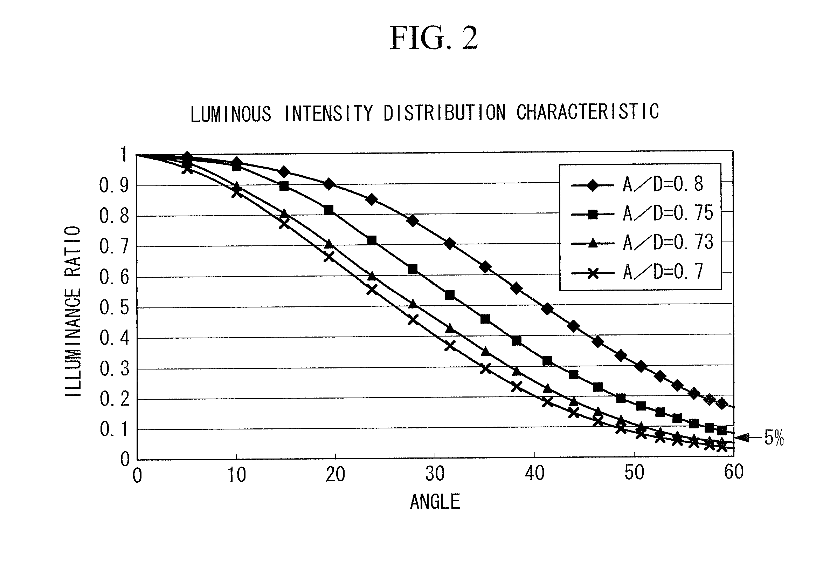

Fig. 2 shows a luminous intensity distribution characteristic (the relationship between an illuminance ratio and an angle) for cases where the values of A/D are 0.8 and 0.75, satisfying the condition (3), and cases where the values are 0.73 and 0.7, which are below the lower limit value of the  condition (3). As shown in Fig. 2, the luminous intensity distribution characteristic is good in the cases of 0.8 and 0.75 where the condition (3) is satisfied, and in contrast, the luminous intensity distribution characteristic is reduced in the cases of 0.73 and 0.7 below the condition (3). Normally, as the lower limit value of the luminous intensity distribution characteristic, the center illuminance of 5% as the illuminance at an angle 60° serves as a rough indication. The reason is that this is the limit for allowing reduction in the brightness around the field of view in an objective optical system with a viewing angle of 120° (a half viewing angle 60°) where a concave lens is adopted as the illumination optical system. The position of the center illuminance of 5% is shown in Fig. 2 with an arrow. According to Fig. 2, the center illuminance is 5% in a case the value of A/D is 0.75, which is the lower limit of the condition (3). Accordingly, the lower limit value of the condition (3) is preferably 0.75 from the viewpoint of the luminous intensity distribution characteristic.

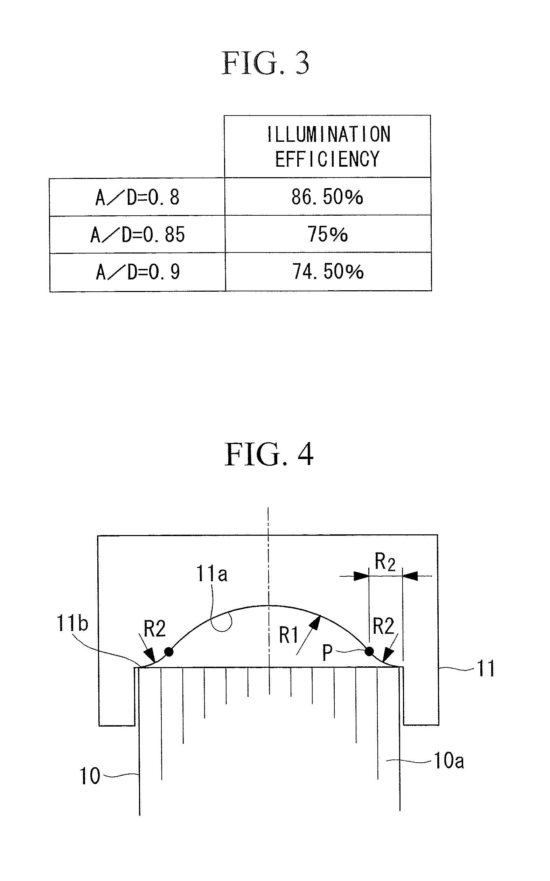

On the other hand, Fig. 3 shows a comparison table of illumination efficiency for cases where the values of A/D are 0.8 and 0.85, satisfying the condition (3), and a case where the value is 0.9, which exceeds the upper limit value of the condition (3).

According to the table in Fig. 3, the illumination efficiency is reduced in a case the illumination lens 11 exceeds the condition (3). When the illumination efficiency falls below 75%, the illumination light hitting the side surface of the illumination lens tends to increase and produce heat such that the temperature at the tip end portion of the endoscope is increased, and thus, the lower limit of the illumination efficiency is preferably 75%. Accordingly, the upper limit value of the condition (3) is preferably 0.85 from the viewpoint of the illumination efficiency.

The upper limit value and the lower limit value of the condition (3) are determined for this reason.

Furthermore, with the illumination lens 11, a curvature radius R1 of the first concave surface 11a and a curvature radius R2 of the second concave surface 11b satisfy the following condition (4).

$$0.25 \leq |R2/R1| \leq 0.35 \quad \ldots (4)$$

In the case where |R2/R1| falls below the condition (4), the area of the second concave surface 11b of the concave surface of the illumination lens 11 becomes relatively small, and the illumination light at the peripheral portion of the light guide bundle is strongly refracted in a direction away from the optical axis and does not reach an observation target, and the illumination efficiency will fall below 75%. Fig. 4 shows an example of a case where |R2/R1| is 0.05, which is below the condition (4).

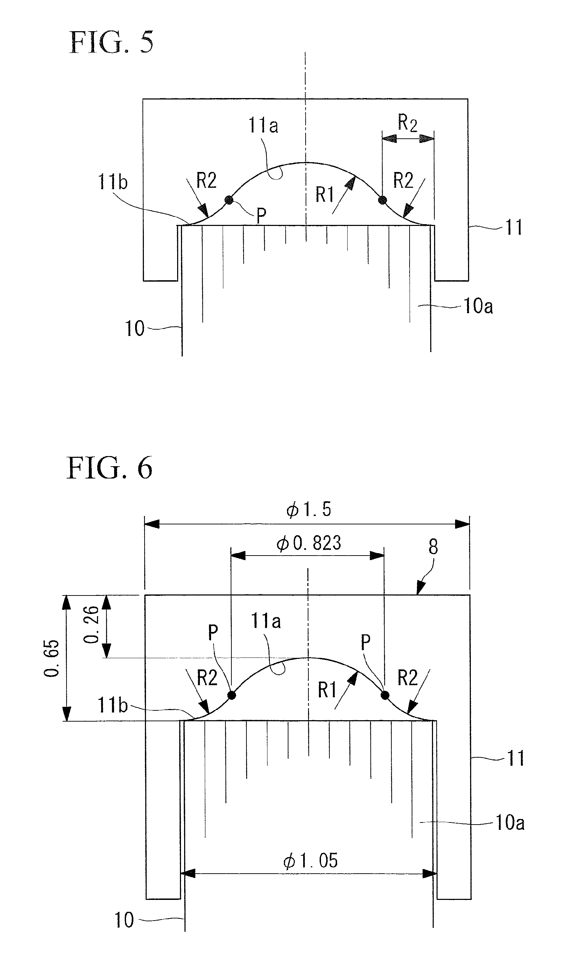

In the case where |R2/R1| exceeds the condition (4), the area of the second concave surface 11b of the concave surface of the illumination lens 11 becomes relatively large, and the illumination light diffused at the first concave surface 11a is reduced, the luminous intensity distribution characteristic is reduced, and the illuminance at an angle 60° falls below the center illuminance of 5%. Fig. 5 shows an example of a case where |R2/R1| is 0.5, exceeding the condition (4). The upper limit value and the lower limit value of the condition (4) are determined for this reason.

Moreover, the illumination lens 11 has the second concave surface 11b arranged, at an outer peripheral edge of the light guide bundle 10, facing an optical axis direction, and efficiently guides the illumination light emitted from a light guide fiber arranged at a peripheral portion of the light guide bundle to the second concave surface 11b.

According to the present invention, illumination light emitted from the light guide fiber 10a located near the center of the light guide bundle 10 is diffused by the first concave surface located in the center of the illumination lens 11. On the other hand, illumination light emitted from the light guide fiber 10a located at a peripheral portion of the light guide bundle 10 is refracted by the second concave surface facing the optical axis direction. The direction of the curve of the second concave surface is opposite to that of the first concave surface with the points of inflection as the boundary, and thus, illumination light incident on the second concave surface is refracted in a direction toward the center of the illumination lens.

Accordingly, illumination light emitted from the light guide fiber 20a located at a peripheral portion of the light guide bundle 10 is also emitted from a tip end surface (an aperture portion) 11c of the illumination lens, and the proportion of illumination light reaching an observation target to the entire illumination light is increased, and sufficient luminous intensity distribution performance may be obtained.

Moreover, the illumination lens 11 includes, in a vertical cross section including the optical axis of the lens 11, the two points of inflection P, the first concave surface 11a that is located on the inner side of the two points of inflection P, and the annular second concave surface 11b that is located on the outer side of the points of inflection P and that is continuous with an outer periphery of the first concave surface 11a, and thus, for example, even if it is formed from an inexpensive glass material with low refractive index or from a material with low refractive index, such as resin, sufficient luminous intensity distribution performance may be achieved.

Furthermore, the tip end portion of an insertion portion of the endoscope and the illumination lens may be double moulded, for example, and in this case, the manufacturing cost may be further lowered.

In the following, concrete examples of the endoscopic illumination optical system according to the embodiment described above will be described as Examples 1 to 5.

(Example 1)

Fig. 6 shows a vertical cross section of an endoscopic illumination optical system according to Example 1 including an optical axis, and according to the endoscopic illumination optical system of Example 1, the diameter dimension D of the end surface of the light guide bundle is 1.05, the curvature radius R1 of the first concave surface 11a is 0.59, the curvature radius R2 of the second concave surface 11b is 0.16, and the distance A between the points of inflection P is 0.823.

(Example 2)

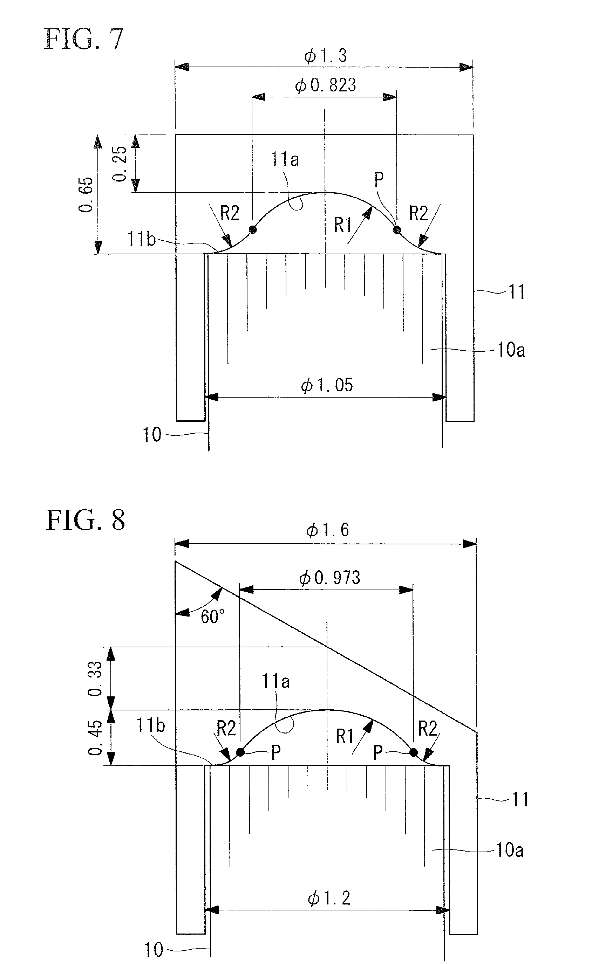

Fig. 7 shows a vertical cross section of an endoscopic illumination optical system according to Example 2 including an optical axis, and according to the endoscopic illumination optical system of Example 2, the diameter dimension D of the end surface of the light guide bundle is 1.05, the curvature radius R1 of the first concave surface 11a is 0.59, the curvature radius R2 of the second concave surface 11b is 0.2, and the distance A between the points of inflection P is 0.823.

(Example 3)

Fig. 8 shows a vertical cross section of an endoscopic illumination optical system according to Example 3 including an optical axis, and according to the endoscopic illumination optical system of Example 3, the diameter dimension D of the end surface of the light guide bundle is 1.2, the curvature radius R1 of the first concave surface 11a is 0.65, the curvature radius R2 of the second concave surface 11b is 0.2, and the distance A between the points of inflection P is 0.973.

(Example 4)

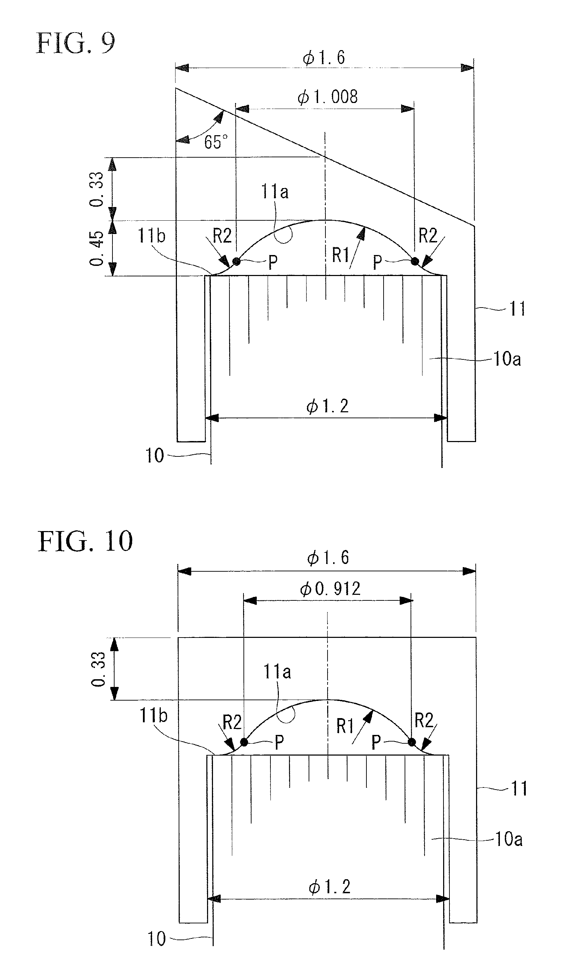

Fig. 9 shows a vertical cross section of an endoscopic illumination optical system according to Example 4 including an optical axis, and according to the endoscopic illumination optical system of Example 4, the diameter dimension D of the end surface of the light guide bundle is 1.2, the curvature radius R1 of the first concave surface 11a is 0.65, the curvature radius R2 of the second concave surface 11b is 0.22, and the distance A between the points of inflection P is 1.008.

(Example 5)

Fig. 10 shows a vertical cross section of an endoscopic illumination optical system according to Example 5 including an optical axis, and according to the endoscopic illumination optical system of Example 5, the diameter dimension D of the end surface of the light guide bundle is 1.2, the curvature radius R1 of the first concave surface 11a is 0.65, the curvature radius R2 of the second concave surface 11b is 0.169, and the distance A between the points of inflection P is 0.912.

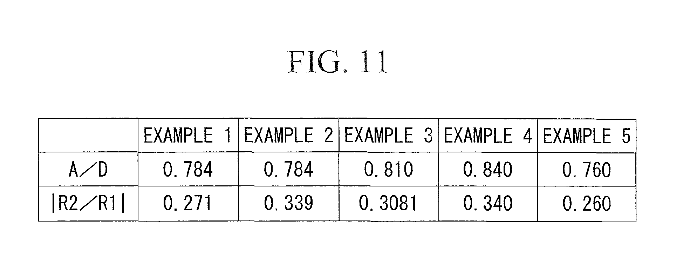

The values of A/D and the values of |R2/R1| of the illumination optical systems for endoscopes according to Examples 1 to 5 described above are shown in Fig. 11. As shown in Fig. 11, Examples 1 to 5 all satisfy the conditions (3) and (4) described above.

{Reference Signs List}

1    endoscopic illumination optical system
10   Light guide bundle
10a  Light guide fiber
11   Illumination lens
11a  First concave surface
11b  Second concave surface

**Claims**

1. An endoscopic illumination optical system, comprising:

    a light guide bundle for guiding illumination light emitted from a light source, the light guide bundle being provided in an insertion portion of an endoscope device; and
    an illumination lens for distributing the illumination light emitted from the light guide bundle to an observation target,
    wherein the illumination lens includes two points of inflection in a vertical cross section including an optical axis of the lens, and includes a first concave surface that is located on an inner side of the two points of inflection, and an annular second concave surface that is located on an outer side of the two points of inflection and that is continuous with an outer periphery of the first concave surface, the second concave surface being arranged so as to face an outer peripheral edge of the light guide bundle in an optical axis direction, and

2. to claim 1,wherein a distance A between the two points of inflection in the vertical cross section including the optical axis and a diameter dimension D of an end surface of the light guide bundle satisfy a following condition (1):

$$0.75 \leq A/D \leq 0.85 \quad ...(1).$$

3. The endoscopic illumination optical system according to claim 1,wherein a curvature radius R1 of the first concave surface and a curvature radius R2 of the second concave surface satisfy a following condition (2):

$$0.25 \leq |R2/R1| \leq 0.35 \quad ...(2).$$

FIG. 1

# FIG. 2

LUMINOUS INTENSITY DISTRIBUTION CHARACTERISTIC

## FIG. 3

|  | ILLUMINATION EFFICIENCY |
|---|---|
| A／D=0.8 | 86.50% |
| A／D=0.85 | 75% |
| A／D=0.9 | 74.50% |

## FIG. 4

FIG. 5

FIG. 6

FIG. 7

FIG. 8

FIG. 9

FIG. 10

FIG. 11

|          | EXAMPLE 1 | EXAMPLE 2 | EXAMPLE 3 | EXAMPLE 4 | EXAMPLE 5 |
|----------|-----------|-----------|-----------|-----------|-----------|
| A／D     | 0. 784    | 0. 784    | 0. 810    | 0. 840    | 0. 760    |
| \|R2／R1\| | 0. 271    | 0. 339    | 0. 3081   | 0. 340    | 0. 260    |

## INTERNATIONAL SEARCH REPORT

| International application No. |
| --- |
| PCT/JP2012/077082 |

**A. CLASSIFICATION OF SUBJECT MATTER**
*G02B23/26*(2006.01)i, *A61B1/00*(2006.01)i

According to International Patent Classification (IPC) or to both national classification and IPC

**B. FIELDS SEARCHED**

Minimum documentation searched (classification system followed by classification symbols)
G02B23/26, A61B1/00

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | JP 64-003616 A (Olympus Optical Co., Ltd.),<br>09 January 1989 (09.01.1989),<br>entire text; all drawings<br>& US 4952040 A | 1<br>2,3 |
| X<br>A | JP 04-142506 A (Olympus Optical Co., Ltd.),<br>15 May 1992 (15.05.1992),<br>page 1, lower right column, line 19 to page 2,<br>upper left column, line 6; fig. 18<br>(Family: none) | 1<br>2,3 |
| A | JP 60-064321 A (Olympus Optical Co., Ltd.),<br>12 April 1985 (12.04.1985),<br>entire text; all drawings<br>& US 4666246 A | 1-3 |

☐ Further documents are listed in the continuation of Box C.     ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 05 December, 2012 (05.12.12) | 18 December, 2012 (18.12.12) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**EP 2 667 235 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI10288742 B